Europäisches Patentamt

(19) European Patent Office

Office européen des brevets

(11) Publication number: **0 279 118 B1**

## (12) EUROPEAN PATENT SPECIFICATION

(45) Date of publication of patent specification: **18.09.91**    (51) Int. Cl.⁵: **A61L  15/16, A61L 25/00**

(21) Application number: **87310644.7**

(22) Date of filing: **03.12.87**

(54) Adhesive products.

(30) Priority: **04.12.86 GB 8629076**

(43) Date of publication of application:
**24.08.88 Bulletin  88/34**

(45) Publication of the grant of the patent:
**18.09.91 Bulletin  91/38**

(84) Designated Contracting States:
**AT BE CH DE ES FR GB IT LI NL SE**

(56) References cited:
**EP-A- 0 063 898**
**EP-A- 0 107 915**
**GB-A- 2 008 000**
**GB-A- 2 094 809**

**CHEMICAL ABSTRACTS, vol. 102, no. 22, 3rd
June 1985, page 402, abstract no. 191207e,
Columbus, Ohio, US; & JP-A-59 232 553
(NITTO ELECTRIC INDUSTRIAL CO., LTD.
SUNSTAR, INC.) 27-12-1984**

(73) Proprietor: **SMITH & NEPHEW plc
2 Temple Place Victoria Embankment
London WC2R 3BP(GB)**

(72) Inventor: **Cunningham, Barry Charles
206 Wymersley Road
Hull North Humberside(GB)**
Inventor: **Shaw, Charles James
59 Randsfield Avenue
Brough Hull North Humberside(GB)**
Inventor: **Robinson, Anthony
7 Beaconsfield Villas Holland Street
Hull North Humberside(GB)**

(74) Representative: **Cole, William Gwyn
Corporate Patents Department Smith and
Nephew Research Limited Gilston Park
Harlow Essex CM20 2RQ(GB)**

## Description

The present invention relates to adhesive products and processes for their preparation.

Adhesive products suitable for use on human skin and wounds such as wound dressings, bandages and ostomy devices often comprise a backing layer and a pressure sensitive adhesive layer. The pressure sensitive adhesive layer of these products can advantageously be a water absorbing layer to enable the adhesive product in use to absorb body fluids such as wound exudate. Adhesive products which employ a water absorbing pressure sensitive adhesive layer are known.

In UK Patent Specification No. 1088992 protective dressings are described comprising a sheet of plastic adhesive in which the adhesive comprises a blend of a water-soluble or water-swellable hydrocolloid and a water insoluble, viscous elastic binder. Such dressings may also be provided with a backing of a water-impervious material.

Other dressings wherein an adhesive layer containing a water-soluble or water-swellable polymer or hydrocolloid is coated onto a foam intermediary layer and the foam is backed with a water-impervious layer are described in UK Patent Specification No. 1548678 and European Patent Application Publication No. 0092999.

A known commercial product of the above type is a wound dressing known as Granuflex which is used on exuding wounds such as ulcers. It has been found, however, that the pressure sensitive adhesive layer of the Granuflex dressing when left in contact with aqueous liquids over long periods of time will tend to disperse or break up.

In UK Patent Application Publication No. 2094809 there is disclosed a pressure sensitive adhesive composition, stated to be useful for application to human skin comprising selected rubbers, selected moisture absorbing components and silica, the adhesive having the capacity to absorb from about 15% to about 40% of its own weight of water. Such adhesives may be employed for the manufacture of surgical sheet materials, bandages and dressings.

Known moisture absorbing materials or hydrocolloids exemplified by the prior art include karaya gum, locust bean gum, guar gum, sodium acrylate, polyvinyl alcohol, pectin, gelatin, carboxymethyl-cellulose, high molecular weight carbowaxes, carboxy polymethylene and collagens.

In UK Patent No. 2008000B there is described a pressure sensitive tape or sheet such as a finely perforated plastic film coated with a layer of an adhesive formed from a water soluble polyol and not more than 25% by weight of a water-soluble or water-swellable polymer which may be sodium alginate. Such tapes or sheets are employed for application to skin without causing skin lesions or irritation of the skin when applied for long periods of time and as such are required to absorb only that water produced by skin respiration.

An adhesive product has now been discovered which has a water absorbent pressure sensitive adhesive layer which will remain coherent when left in contact with aqueous liquids, such as wound exudate for long periods of time.

Accordingly the present invention provides an adhesive product suitable for use on wounds which comprises a backing layer of a moisture vapour transmitting continuous polymer film and a layer of a pressure sensitive adhesive containing at least 30% by weight of acrylic adhesive comprising alkyl acrylate residues and at least 30% by weight of alginate.

The alginate in the pressure sensitive adhesive layer renders the layer water absorbable.

Suitable alginates for use in the invention include both water insoluble and water soluble alginates. Preferred alginates for use in the invention, however, are water soluble alginates. Such water soluble alginates can render the pressure sensitive adhesive layer highly water absorbable. A favoured water soluble alginate for use in the invention is sodium alginate. The sodium alginate, however, can advantageously contain up to 1.5% by weight of a calcium.

An apt sodium alginate for use in the invention is known as Manucol DM available for Kelco AIL.

The acrylic adhesive used in the invention renders the adhesive layer pressure sensitive. The acrylic adhesive used in the invention can be any of the acrylic adhesives comprising alkyl acrylate residues used in a conventional surgical or medical tapes, bandages, wound dressings and ostomy devices. Such acrylic adhesives usually comprise alkyl acrylate residues in which the alkyl group contains 4 to 10 carbon atoms for example n-butyl acrylate and 2-ethylhexyl acrylate. The acrylic adhesive can also comprise acrylic acid residues to improve the cohesive properties of the adhesive.

Favoured acrylic adhesives for use in the invention which comprise n-butyl acrylate residues, 2-ethylhexyl acrylate residues and acrylic acid residues are disclosed in European Patent No. 35399.

Suitable acrylic adhesives include both hot melt and solution coatable acrylic adhesives.

An apt solution coatable acrylic adhesive for use in the invention which comprises 47 parts by weight of n-butyl acrylate residues, 47 parts by weight of 2-ethylhexyl acrylate residues and 6 parts by weight of acrylic acid residues is disclosed in Example 1 of European Patent No. 0035399.

An apt hot melt coatable adhesive for use in the invention is known as Synthacryl VS6 9227 available from Resinous Chemicals Ltd.

The pressure sensitive adhesive layer used in the invention will contain at least 30% by weight of acrylic adhesive and at least 30% by weight of alginate. The pressure sensitive adhesive layer therefore can contain 30% to 70% by weight of acrylic adhesive and 30% to 70% by weight of alginate.

It has been found that an amount of 30% by weight is close to the minimum amount of alginate which will provide the adhesive layer with adequate absorption properties. It has also been found that an amount of alginate in excess of 70% by weight will normally render the adhesive layer non conformable and unsuitable for adhering to skin.

It is desirable, however, that the adhesive layer has relatively high absorption properties and adequate adhesive properties.

Pressure sensitive adhesive layers for use in the invention desirably contain at least 35% by weight, more desirably at least 45% by weight and preferably at least 55% by weight of alginate for example 60% to 65% by weight.

Favoured adhesive layers for use in the invention contain 55% to 65% by weight of alginate and 35% to 45% of acrylic adhesive.

Apt adhesive layers of this type contain 60% to 62% by weight of alginate and 38% to 40% by weight of acrylic adhesive. The adhesive layer, however, can optionally contain other materials such as fillers, preservatives, antioxidants or medicaments.

The pressure sensitive adhesive layer used in the invention can suitably have a thickness of 0.5mm to 2.5mm, and can preferably have a thickness of 1mm to 2mm for example a thickness 1.5mm to 1.8mm.

The use of such thick pressure sensitive adhesive layers in the adhesive product of the invention provides the adhesive product in use with a high capacity to absorb aqueous fluids such as wound exudate.

The pressure sensitive adhesive layer used in the invention swells on contact with water or aqueous fluids such as wound exudate or a physiological saline solution. It has been found, however, that the pressure sensitive adhesive layer of the adhesive product, although swollen, remains coherent and intact even when the adhesive products exposed to such aqueous fluids for time periods up to 192 hours.

The pressure sensitive adhesive layer used in the invention can be a continuous or discontinuous layer for example a porous layer . It is preferred, however, that the pressure sensitive adhesive layer is a continuous layer.

The backing layer used in the invention comprises a moisture vapour transmitting continuous polymer film. The continuous polymer film used in the invention does not have openings such as holes, pores or micropores which extend completely through the film. The continuous polymer film used in the invention thus does not have any openings or pores which can provide a passageway for liquid water and bacteria through the film. Such a film therefore can act as a barrier to liquid water or aqueous fluids and bacteria penetrating from the outside of a wound dressing of invention in use to the wound site or vice versa. The backing layer will be conformable to render the adhesive product conformable.

The continuous polymer film used in the invention is moisture vapour transmitting. The moisture vapour transmission rate of the film used in the invention can be determined by the Payne Cup Method described in European Patent No. 107915 in which the cup is inverted so that the film sample is in contact with water. This "wet" moisture vapour transmission rate is expressed herein as $g/m^2/24$ hours at $37°C$ at a relative humidity difference of 100% to 10%, abbreviated hereinafter as $g/m^2$.

The continuous polymer film used in the invention film desirably has a moisture vapour transmission rate which is sufficient to permit evaporation of absorbed aqueous body fluid from the pressure sensitive adhesive layer of the adhesive product of the invention in use thereby increasing the absorbent capacity of the adhesive product. The continuous polymer film used in the invention can suitably have a "wet" moisture vapour transmission rate of at least 1000 $g/m^2$, desirably of at least 2000 $g/m^2$ and can preferably have a "wet" moisture vapour transmission rate of at least 5000 $g/m^2$ for example 7000 $g/m^2$.

The continuous polymer film used in the invention can suitably have a thickness of 10 to 80$\mu$m, and can preferably have a thickness of 15 to 65$\mu$m.

The polymer used for the continuous film is preferably an elastomer. A backing layer of continuous elastomer film has the advantage of rendering the adhesive product of the invention highly comformable. The elastomer is preferably also a linear elastomer. Linear elastomer films can advantageously be formed by a conventional solvent or hot melt casting technique. Suitable linear elastomers for the continuous film used in the invention include both hydrophilic and relatively non-hydrophilic elastomers such as polyurethanes, polyether-amide and polyester ether elastomers. Suitably continuous films of these

3

EP 0 279 118 B1

elastomers include those disclosed in the aforementioned European Patent No. 107916.

Suitable relatively non-hydrophilic polyurethane elastomers for use in the film used in the invention include linear polyester polyurethane and linear polyether polyurethane elastomers for example those known as Estanes available from BF Goodrich (UK) Limited.

Apt grades are Estane 5701, 5702, 5714 and 58201. An apt film for use in the invention is a 25$\mu$m thick polyether polyurethane (Estane 5714) continuous film which has a "wet" moisture vapour transmission rate of 1800 to 2000 g/m$^2$.

The hydrophilic linear polyurethane elastomers for use in the invention can suitably have a water content when hydrated from 5% to 50% by weight, desirably from 10% to 40% by weight and can preferably have a water content when hydrated from 20% to 30% by weight for example 25% by weight. Suitable hydrophilic linear polyurethane elastomers for use in the invention are described in United Kingdom Patent No. 2093190. An apt hydrophilic linear polyurethane elastomer for use in the invention which has water content when hydrated of 25% by weight is described in Example 2 of the United Kingdom Patent No. 2093190. An apt film of this hydrophilic linear polyurethane elastomer for use in the invention has a weight per unit area of approximately 20 g/m$^2$ and a "wet" moisture vapour transmission rate of >14000g/m$^2$.

The use of a hydrophilic polymer film such as a hydrophilic polyurethane film as the backing layer of the adhesive product of the invention can be advantageous as will be explained hereinafter.

Suitable polyether-amide elastomers are disclosed in British Patent 1473972 French Patent Nos. 1444437 and 2178205 and United States Patent No.3839245. An apt polyether-amide is known as Pebax 4011 RNOO available from ATO Chemical Products (UK) Limited. This polymer has a water content of approximately 55% when hydrated. An apt film of Pebax 4011 RNOO for use in the invention has a thickness of 70$\mu$m and a wet moisture vapour transmission rate of >14000 g/m$^2$.

Suitable polyether-ester elastomers for use in the invention are known as Hytrel available from Du Pont (UK) Limited. An apt grade is known as Hytrel 4056. An apt film of Hytrel 4056 for use in the invention has a thickness of 70$\mu$m and a wet moisture vapour transmission rate of 4,200g/m$^2$.

The adhesive product of the invention has a pressure sensitive adhesive layer which is capable in use of absorbing aqueous body fluids such as wound exudate. Desirably the adhesive product is capable of absorbing at least 50% by weight and preferably at least 100% by weight of fluid when immersed in a physiological saline solution (0.9% by weight% at 20$^\circ$C for 24 hours. It has been found that adhesive products of the invention can absorb relatively large amounts of fluid for example over 500% by weight when left immersed in the saline solution for long periods for example 192 hours. Furthermore, it has been found that adhesive products of the invention remain intact and do not break up during these long periods of immersion. Adhesive products of the invention thus have an advantage over the prior art Granuflex product which tends to break up when immersed in saline solution for long periods of time. Adhesive products of the invention which comprise a hydrophilic polymer backing film have a further advantage in that the adhesive product does not 'curl' when immersed in aqueous fluids. It is believed that this advantage stems from the use of a backing layer which can swell at a similar rate to that of the adhesive layer.

The combination of a water absorbing pressure sensitive adhesive layer and a moisture vapour transmitting backing layer in the adhesive product of the invention allows the adhesive product when wet to be moisture vapour transmitting. The adhesive product of the invention can suitably have a "wet" moisture vapour transmitting rate of at least 500 g/m$^2$ and can preferably have a "wet" moisture vapour transmitting of at least 750 g/m$^2$ for example 1000 to 1200 g/m$^2$. The adhesive product will normally be provided with a protective layer such as a release coated film or paper over the pressure sensitive adhesive layer of the product. which can be removed prior to use.

The adhesive product will preferably be sterile within a bacteriaproof pack for example a sealed pouch comprising for example a conventional paper - plastics film laminate or a paper aluminium foil - plastic film laminate.

The adhesive product can conveniently be sterilised within the pack by a conventional sterilising method including gamma and electron irradiation and ethylene oxide gas.

The adhesive product of the invention can contain a medicament which will normally be present in the adhesive layer of the product. The medicament will preferably be a medicament for topical use such as a chlorhexidine derivative. However, the medicament may form part of the alginate polymer used for the pressure adhesive layer for example a water soluble silver, copper or zinc alginate derivative of sodium alginate. Alternatively, the medicament can be a water insoluble silver, copper or zinc alginate dispersed within the pressure sensitive adhesive layer. Such water insoluble alginates can advantageously provide a sustained release of antibacterial metal ions into the wound site of an exuding wound.

The adhesive product of the invention is advantageously both conformable and moisture vapour

4

transmitting. In addition, the adhesive product has a continuous film backing layer which acts as a barrier to bacteria and liquid water and an adhesive layer which can absorb and retain aqueous fluids without leakage, but remain intact and coherent when exposed thereto for long periods.

For these reason the adhesive product of the invention will be suitable for use on wounds, and in particular highly exuding wounds such as ulcers.

Therefore in another aspect the invention provides a wound dressing which comprises a backing layer of a moisture vapour transmitting continuous polymer film and a layer of pressure sensitive adhesive containing at least 30% by weight of acrylic adhesive comprising alkyl acrylate residues and at least 30% by weight of alginate.

The adhesive product can also suitably be for use with ostomy wounds. The adhesive product therefore can also be an ostomy device for example a colostomy bag attachment ring.

A wound can be treated by a method which includes applying to the wound a wound dressing which comprises a backing layer of a moisture vapour transmitting continuous polymer film and a layer of pressure sensitive adhesive containing at least 30% by weight of acrylic adhesive and at least 30% by weight of alginate.

The pressure sensitive adhesive layer of the adhesive product such as a wound dressing enables the adhesive product to be adhered to the intact skin and in particular to moist skin around a wound site and to cover the wound site. The combination of the absorption and vapour transmission properties of the adhesive product maintains the skin around the wound site very healthy and also reduces the tendency of exudate to leak from the sides of the product.

The size and shape of intended use of the adhesive product can be adapted for its intended use. Substantially square and rectangular shapes of 10cm x 10cm, 15cm x 20cm, 20cm x 20cm sizes have been found suitable for wound dressings.

In another aspect the invention provides a process for the preparation of an adhesive product of the invention which comprises coating a pressure sensitive layer onto a backing layer characterised in that the backing layer comprises a moisture vapour transmitting continuous polymer film and the pressure sensitive adhesive layer comprises at least 30% by weight of acrylic adhesive comprising alkyl acrylate residues and at least 30% by weight of alginate.

The materials used in the process of the invention can be the same as used in the adhesive product of the invention.

The backing layer for use in the process of the invention can conveniently be formed by casting the polymer as continuous film onto a release coated substrate such as silicone release coated paper or film using a conventional solution or hot melt extrusion technique as appropriate.

The adhesive for the pressure sensitive adhesive layer can be formed by mixing the alginate into acrylic adhesive. The alginate for example as fine powder can be mixed into a solution or hot melt of the acrylic adhesive by a conventional technique. The adhesive mixture can then be coated directly or indirectly onto the backing layer and dried in an oven to form the adhesive product of the invention.

In a preferred process of the invention the adhesive mixture is first coated onto release substrate by a conventional coating technique such as a knife over flat bed solution coating or a hot melt coating technique, dried (if necessary) and then laminated to the backing layer on its release substrate by passing the combined layers through the nip of a pair of pressure rollers. The release substrate can then be removed from the backing layer to form the adhesive product of the invention. The adhesive product can then be cut into the appropriate size, packed into a bacteriaproof pack and sterilised by a conventional method such as gamma irradiation, electron irradiation or ethylene oxide gas.

The invention will now be illustrated by reference to the following example.

## Example 1

### Preparation of adhesive wound dressing

### Backing layer

A solution (15% by weight) industrial methylated spirits of hydrophilic polyurethane (having a water content of 25% by weight when hydrated) prepared according to example 2 of United Kingdom Patent No. 2093190 or European Patent No. 5035 containing 5% by weight of fine silica was cast onto a silicone coated release paper and dried to give a film having a weight per unit area of 20 g/m$^2$.

### Pressure sensitive adhesive layer

33.8 parts by weight of sodium alginate powder (Manucol DM) was added to 66.2 parts of by weight of a solution (32% by weight) in acetone of a polyacrylate adhesive prepared according to Example 2 of European Patent No. 35399 (contains residues of 47 parts by weight n-butyl acrylate, 47 parts by weight 2-ethylhexyl acrylate and 6 parts by weight acrylic acid) and mixed thoroughly. The mixture was then cast onto a silicone release paper using a conventional knife over flat bed technique and dried in an oven for approximately 5 hours at temperatures from 30°C to 100°C to provide a dried layer with a thickness of approximately 1.5mm. The dried pressure sensitive adhesive layer so formed comprised 38.5% by weight acrylic adhesive and 61.5% by weight sodium alginate.

The backing layer was then transfer laminated to the adhesive layer by passing the two layers on their release papers through the nip of two pressure rollers and removing the release paper from the backing layer. The laminate was then cut in 10cm × 10cm pieces to form adhesive dressings of the invention. Individual dressings were then packed in paper - aluminium foil sealed packs and sterilised by gamma irradiation.

The adhesive dressings (release paper removed) had a "wet" moisture vapour transmission rate of $1167 \pm 44$ g/m$^2$ (test method according to page 52 of European Patent 107915 using an inverted Payne cup in which the adhesive layer contacted the water in the cup).

In adhesive tests the dressings were found to adhere well to both dry and wet skin.

Absorbency test

10cm × 10cm adhesive dressings prepared according to the example and comparison 10cm × 10cm commercially available Granuflex dressings were tested for absorbency by immersing the dressings in a physiological saline solution (0.9% by weight in water) at 20°C.

The amount of saline (g) absorbed by individual dressings was measured after 24, 48, 120 and 192 hours immersion and the amount expressed as a weight % of the weight of the dressing.

The results of the test were as follows:

## The results of the test were as follows:

| | Immersion Time (hours) | Saline absorbed (grams) | Saline absorbed by the dressing (% by wt) |
|---|---|---|---|
| Dressing | 24 | 27.1 | 218 |
| of example | 48 | 49.8 | 402 |
| | 120 | 61.8 | 496 |
| | 192 | 63.8 | 512 |
| Comparison | 24 | 17.0 | 100 |
| Dressing | 48 | 29.8 | 185 |
| | 120 | 55.8 | 327 |
| | 192 | Dressing disintegrated | |

The test results showed that the adhesive dressing of the invention absorbed a higher amount of a saline and at higher rate than the comparison prior art commercial Granuflex dressing. Furthermore the test results showed that adhesive dressing of the invention remained intact during contact with saline for 192 hours whereas the comparison Granuflex dressing disintegrated during the same time period. It was also found during the test that the adhesive dressing of the invention did not curl or exhibited slight curling at the edges during immersion in saline whereas the comparison Granuflex dressing exhibited severe curling during immersion.

Example 2

Preparation of Adhesive Wound Dressing

Backing Layer

The backing layer used in the dressing of this example was the same and was prepared in the same manner on release coated paper as that of Example 1.

Pressure Sensitive Adhesive Layer

40 parts by weight of a hot melt pressure sensitive adhesive (Synthacryl VSC 9227) and 60 parts by weight of sodium alginate powder were added to a 2-blade mixer heated by circulating oil to a temperature of 70 to 75°C and mixed for approximately 40 mins to form a homogenous blend of the two components.

The adhesive was then extruded using an extruder with slit die (die temperature 70-75°C) as a layer (1.5mm thick) onto a silicone release coated paper. The backing layer on its carrier was then laminated to the adhesive layer by pressing the layers through the nip of a pair of pressure rollers. The carrier was then removed from the backing layer and the laminated sheet cut into 10cm × 10cm waifers to form adhesive dressings of the invention.

The adhesive dressings (release paper removed) had a "wet" moisture vapour transmission rate of 818 g/m$^2$ (test method according to European Patent 107915).

Absorbency Test

An absorbency test was carried out on the adhesive dressing of Example 2 in the same manner as Example 1. The results of the test were as follows:

| Immersion Time (hours) | Saline absorbed by the dressing | |
|---|---|---|
| | (grams) | (% by wt) |
| 24 | 83.2 | 327 |
| 72 | 129.2 | 433 |
| 96 | 149.0 | 486 |

The test results thus indicate that the adhesive dressings of the invention prepared according to the above Example have properties which render the dressings highly suitable for use on heavily exuding wounds such as ulcers.

**Claims**

1.  An adhesive product suitable for use on wounds which comprises a backing layer of a moisture vapour transmitting continuous polymer film and a layer of pressure sensitive adhesive containing at least 30% by weight of acrylic adhesive comprising alkyl acrylate residues and at least 30% by weight of alginate.

2.  An adhesive product as claimed in claim 1 in which the adhesive contains 30% to 70% by weight of acrylic adhesive and 30% to 70% by weight of alginate.

3. An adhesive product as claimed in either of claims 1 or 2 in which adhesive contains at least 55% by weight of alginate.

4. An adhesive product as claimed in any of claims 1 to 3 in which the alginate is sodium alginate.

5. An adhesive product as claimed in any of claims 1 to 4 in which the adhesive layer is continuous and has a thickness of 1mm to 2mm.

6. An adhesive product as claimed in any of claims 1 to 5 in which the continuous polymer film has a thickness of 15 to 65$\mu$m and a wet moisture vapour transmission rate at least 2000 g/m$^2$.

7. An adhesive product as claimed in any of claims 1 to 6 in which the continuous film polymer is a hydrophilic linear polyurethane elastomer which has a water content when hydrated of 10% to 40% by weight.

8. An adhesive product as claimed in any of claims 1 to 7 which has a fluid absorbent capacity when hydrated of at least 100% by weight.

9. An adhesive product as claimed in any of claims 1 to 8 which has a wet moisture vapour transmission rate of at least 750 g/m$^2$.

10. An adhesive product as claimed in any of claims 2 to 9 which contains a topical medicament.

11. An adhesive product as claimed in any of claims 1 to 10 which is sterile within a bacteriaproof pack.

12. A wound dressing which comprises a backing layer of a moisture vapour transmitting continuous polymer film and a layer of pressure sensitive adhesive containing at least 30% by weight of acrylic adhesive comprising alkyl acrylate residues and at least 30% by weight of alginate.

13. A process for the preparation of an adhesive product as claimed in any of claims 1 to 11 which comprises coating a layer of pressure sensitive adhesive onto a backing layer wherein the backing layer comprises a moisture vapour transmitting continuous polymer film and the pressure sensitive adhesive layer contains at least 30% by weight of acrylic adhesive comprising alkyl acrylate residues and at least 30% by weight of alginate.

14. A process as claimed in claim 13 in which the layer of pressure sensitive adhesive is indirectly coated onto the backing layer by coating the layer onto a release substrate and laminating the layer on the release substrate to the backing layer.

15. A process as claimed in either of claims 13 or 14 in which the pressure sensitive adhesive layer is coated as a hot melt.

16. A process as claimed in any of claims 13 to 15 in which the pressure sensitive adhesive is formed by mixing the alginate into the acrylic adhesive.

**Revendications**

1. Produit adhésif utilisable sur des plaies, caractérisé en ce qu'il comprend une couche de support d'un film de polymère continu transmettant la vapeur d'eau et une couche d'adhésif sensible à la pression contenant au moins 30 % en poids d'adhésif acrylique comprenant des résidus d'acrylate d'alkyle et au moins 30 % en poids d'alginate.

2. Produit adhésif suivant la revendication 1, caractérisé en ce que l'adhésif contient 30 à 70 % en poids d'adhésif acrylique et 30 à 70 % en poids d'alginate.

3. Produit adhésif suivant l'une quelconque des revendications 1 ou 2, caractérisé en ce que l'adhésif contient au moins 55 % en poids d'alginate.

4. Produit adhésif suivant l'une quelconque des revendications 1 à 3, caractérisé en ce que l'alginate est de l'alginate de sodium.

5. Produit adhésif suivant l'une quelconque des revendications 1 à 4, caractérisé en ce que la couche adhésive est continue et a une épaisseur de 1 mm à 2 mm.

6. Produit adhésif suivant l'une quelconque des revendications 1 à 5, caractérisé en ce que le film de polymère continu à une épaisseur de 15 à 65 $\mu$m et un taux de transmission de vapeur d'eau "humide" d'au moins 2000 g/m$^2$.

7. Produit adhésif suivant l'une quelconque des revendications 1 à 6, caractérisé en ce que le polymère du film continu est un élastomère de type polyuréthane linéaire hydrophile qui a une teneur en eau de 10 % à 40 % en poids lorsqu'il est hydraté.

8. Produit adhésif suivant l'une quelconque des revendications 1 à 7, caractérisé en ce qu'il a une capacité d'absorption de fluide d'au moins 100 % en poids lorsqu'il est hydraté.

9. Produit adhésif suivant l'une quelconque des revendications 1 à 8, caractérisé en ce qu'il a un taux de transmission de vapeur d'eau "humide" d'au moins 750 g/m$^2$.

10. Produit adhésif suivant l'une quelconque des revendications 2 à 9, caractérisé en ce qu'il contient un médicament topique.

11. Produit adhésif suivant l'une quelconque des revendications 1 à 10, caractérisé en ce qu'il est stérile à l'intérieur d'un sachet étanche aux bactéries.

12. Pansement pour plaie, caractérisé en ce qu'il comprend une couche de support d'un film de polymère continu transmettant la vapeur d'eau et une couche d'adhésif sensible à la pression contenant au moins 30 % en poids d'adhésif acrylique comprenant des résidus d'acrylate d'alkyle et au moins 30 % en poids d'alginate.

13. Procédé pour la préparation d'un produit adhésif suivant l'une quelconque des revendications 1 à 11, qui comprend l'enduction d'une couche d'adhésif sensible à la pression sur une couche de support, caractérisé en ce que la couche de support comprend un film de polymère continu transmettant la vapeur d'eau et que la couche adhésive sensible à la pression contient au moins 30 % en poids d'adhésif acrylique comprenant des résidus d'acrylate d'alkyle et au moins 30 % en poids d'alginate.

14. Procédé suivant la revendication 13, caractérisé en ce que la couche d'adhésif sensible à la pression est déposée indirectement sur la couche de support par enduction de la couche sur un substrat détachable et feuilletage de la couche avec son substrat détachable sur la couche de support.

15. Procédé suivant l'une quelconque des revendications 13 ou 14, caractérisé en ce que la couche adhésive sensible à la pression est déposée sous forme de masse fondue.

16. Procédé suivant l'une quelconque des revendications 13 à 15, caractérisé en ce que l'adhésif sensible à la pression est formé par mélange de l'alginate dans l'adhésif acrylique.

**Patentansprüche**

1. Für die Verwendung auf Wunden geeignetes selbstklebendes Produkt, welches eine Tragschicht eines für Wasserdampf durchlässigen kontinuierlichen Polymerfilms und eine selbstklebende Schicht mit wenigstens 30 Gew. -% eines Akrylklebers, der Alkylakrylat-Reste und wenigstens 30 Gew. -% Alginat enthält.

2. Selbstklebendes Produkt nach Anspruch 1, bei welchem der Kleber 30 - 70 Gew. -% Akrylkleber und 30 - 70 Gew.-% Alginat enthält.

3. Selbstklebendes Produkt nach Anspruch 1 oder 2, bei welchem der Klebstoff wenigstens 55 Gew. -%

Alginat enthält.

4. Selbstklebendes Produkt nach einem der Ansprüche 1 - 3, bei welchem das Alginat Natriumalginat ist.

5. Selbstklebendes Produkt nach einem der Ansprüche 1 - 4, bei welchem die Klebstoffschicht kontinuierlich ist und eine Dicke von 1mm - 2 mm aufweist.

6. Selbstklebendes Produkt nach einem der Ansprüche 1 - 5, bei welchem der kontinuierliche Polymerfilm eine Dicke Von 15 - 65 $\mu$m und eine Wasserdampfdurchlässigkeit von wenigstens 2000 g/m$^2$ aufweist.

7. Selbstklebendes Produkt nach einem der Ansprüche 1 - 6, in welchem das Polymer des kontinuierlichen Films ein hydrophiles lineares Polyurethan-Elastomer ist, das in hydratisiertem Zustand einen Wassergehalt von 10 Gew. -% - 40 Gew. -% aufweist.

8. Selbstklebendes Produkt nach einem der Ansprüche 1 - 7, welches eine Flüssigkeit-Absorptionskapazität in hydratisiertem Zustand von wenigstens 100 Gew. -% aufweist.

9. Selbstklebendes Produkt nach einem der Ansprüche 1 - 8, welches eine Wasserdampfdurchlässigkeit von wenigstens 750 g/m$^2$ aufweist.

10. Selbstklebendes Produkt nach einem der Ansprüche 2 - 9, dadurch gekennzeichnet, daß es ein äußerlich anwendbares Medikament enthält.

11. Selbstklebendes Produkt nach einem der Ansprüche 1 - 10, welches sich steril innerhalb einer bakteriendichten Verpackung befindet.

12. Wundverband mit einer Stützschicht aus einem wasserdampfdurchlässigen kontinuierlichem Polymerfilm und einer selbstklebenden Schicht mit wenigstens 30 Gew. - % eines Akrylklebers mit Alkylakrylat-Resten und wenigstens 30 Gew. -% Alginat.

13. Ein Verfahren zum Herstellen eines selbstklebenden Produktes gemäß einem der Ansprüche 1 - 11, welches das Aufbringen einer Schicht aus selbstklebendem Klebstoff auf eine Tragschicht einschließt, wobei die Tragschicht einen wasserdampfdurchlässigen kontinuierlichen Polymerfilm aufweist und die selbstklebende Klebstoffschicht wenigstens 30 Gew. -% eines Akrylklebers mit Alkylakrylat-Resten und wenigstens 30 Gew. -% Alginat enthält.

14. Verfahren nach Anspruch 13, bei welchem die Schicht aus selbstklebendem Klebstoff indirekt auf die Tragschicht aufgebracht wird, indem die Schicht auf eine Ablöseschicht aufgebracht und die Schicht mit der Ablöseschicht auf die Tragschicht laminiert wird.

15. Verfahren nach einem der Ansprüche 13 oder 14, in welchem die Schicht aus selbstklebendem Klebstoff als heiße Schmelze aufgebracht wird.

16. Verfahren nach einem der Ansprüche 13 - 15, bei welchem der Selbstkleber durch Einmischen des Alginats in den Akrylkleber hergestellt wird.